**Europäisches Patentamt**

**European Patent Office**

**Office européen des brevets**

(11) Veröffentlichungsnummer: **0 025 841 B1**

# (12) EUROPÄISCHE PATENTSCHRIFT

(45) Veröffentlichungstag der Patentschrift:
**23.02.83**

(21) Anmeldenummer: **80104499.1**

(22) Anmeldetag: **30.07.80**

(51) Int. Cl.³: **C 07 C 59/245**, C 07 B 19/00

(54) Diastereomere Salze von Äpfelsäure und 2-Amino-1-butanol sowie Verfahren zur Spaltung racemischer Äpfelsäure.

(30) Priorität: **22.08.79 DE 2933895**

(43) Veröffentlichungstag der Anmeldung:
**01.04.81 Patentblatt 81/13**

(45) Bekanntmachung des Hinweises auf die Patenterteilung:
**23.02.83 Patentblatt 83/8**

(84) Benannte Vertragsstaaten:
**BE DE FR GB IT NL**

(56) Entgegenhaltungen:
**EP-A-0 000 518**

,,Chemisches Zentralblatt'' Bd. 96, Nr. 6 (1925) II, Berlin A.W. Ingersoll ,,Eine Methode zur vollständigen gegenseitigen optischen Spaltung von racemischen Säuren und Basen'', S. 551 bis 552
,,Chemisches Zentralblatt'', Bd. 110, Nr. 20 (1939) II, Berlin H.L. Dickison *et al.* ,,Die terpenoiden Amine. I. Die isomeren Thujylamine'', S. 3423 bis 3424
,,Chemisches Zentralblatt'', Bd. 131, Nr. 14 (1960), Berlin H. Katsura ,,Stereochemische Untersuchungen über alpha-Hydroxysäuren. 1. Mitt. Optische Spaltung von alpha-Hydroxysäuren'', S. 4482

(73) Patentinhaber: **BASF Aktiengesellschaft,
Carl-Bosch-Strasse 38, D-6700 Ludwigshafen (DE)**

(72) Erfinder: **Berning, Wilfried, Dr., Langstrasse 23,
D-6719 Kirchheimbolanden (DE)**
Erfinder: **Siegel, Hardo, Dr., Hans-Purrmann-Allee 25,
D-6720 Speyer (DE)**

Diastereomere Salze von Äpfelsäure und 2-Amino-1-butanol sowie Verfahren zur Spaltung racemischer Äpfelsäure

Die Erfindung betrifft ein Verfahren zur Spaltung racemischer Äpfelsäure. Insbesondere wird L(−)-Äpfelsäure zugänglich.

Äpfelsäure wird in grossen Mengen in der Lebensmittelindustrie als charakteristisches Säuerungsmittel eingesetzt. Dabei findet vor allem die leicht zugängliche synthetische D,L(−)-Äpfelsäure Verwendung. Die natürliche L(−)-Äpfelsäure lässt sich dagegen nur mit grossem Aufwand über ihre schwerlöslichen Calcium-Salze aus geeigneten Früchten isolieren.

Trotz des erheblichen Preisunterschieds wird ein ständig steigender Bedarf an optisch aktiver Äpfelsäure beobachtet, entsprechend der allgemeinen Entwicklung, nur solche Lebensmittelzusätze zu verwenden, die in natürlichen Lebensmitteln schon vorkommen. Dabei kommt einer neueren Untersuchung des chemisch verwandten Systems Weinsäure/Traubensäure grosse Bedeutung zu [(,,Toxikology" 8, 263-274 und 333-346 (1977)], bei der man eine unterschiedliche Toxizität der optisch aktiven natürlichen und der racemischen synthetischen Form beobachtet hat.

Die Racematspaltung von D,L-Äpfelsäure mit der Base Cinchonin wird in der Literatur beschrieben (G.J.W. Bremer, Recueil 4, 181 (1885). Nach zahlreichen Umkristallisationen wird L(−)-Äpfelsäure in schlechter optischer Reinheit erhalten.

Auch (+)-Phenyläthylamin wurde schon zur Antipodentrennung von D,L-Äpfelsäure verwendet [(B. Holmberg, ,,Z. Phys. Chem." 137, 21 (1928)]. Die schlechte optische Reinheit der Malate machte eine wiederholte Reinigung durch Umkristallisation nötig. Die Ausbeute lag bei 62%.

Den bekannten Racematspaltungen durch fraktionierte Kristallisation diastereomerer Malate ist gemeinsam, dass die eingesetzten Basen sehr teuer sind und dass, bedingt durch die hohen Molekulargewichte, der Einsatz grosser Mengen notwendig wird.

Es bestand daher die Aufgabe, für die Racematspaltung von D,L-Äpfelsäure ein Verfahren auf der Grundlage eines billigen Spaltreagenzes mit möglichst niedrigem Molgewicht und hohem Wirkungsgrad bereitzustellen.

Billige Spaltreagenzien sind z.B. niedere Aminoalkohole, sofern sie ein Chiralitätszentrum besitzen. So wurde die Spaltung racemischer Mandelsäure mit D(−)-2-Amino-1-butanol schon beschrieben (EP-A Nr. 0000518).

Vom Racematgemisch der Äpfelsäure ist jedoch bekannt, dass seine Spaltung mit Hilfe von 1-Amino-2-propanol nicht möglich ist (US-PS Nr. 3116332). Um so überraschender ist die Tatsache, dass diese Antipodentrennung unter Verwendung des nächst höheren Aminoalkohols gelingt.

Es wurde gefunden, dass D,L-Äpfelsäure vorzugsweise in einem niedermolekularen Alkohol mit einem Wassergehalt von unter 40% mit — z.B. — R(−)-2-Amino-1-butanol ein schwerlösliches, auskristallisierendes diastereomeres Salz bildet, das von der Mutterlauge abgetrennt werden kann. Aus den in Kristallisat und Mutterlauge enthaltenen diastereomeren Salzen können mit Säuren und Basen nach an sich bekannten Methoden jeweils die optisch aktiven Säuren und R(−)-2-Amino-1-butanol gewonnen werden.

Optisch aktives R(−)-2-Amino-1-butanol lässt sich z.B. nach der DE-PS Nr. 1243206 leicht aus dem racemischen 2-Amino-1-butanol durch Racematspaltung mit natürlicher (+)-Weinsäure gewinnen. Es zeigt eine hohe Racemisierungsbeständigkeit und kann ohne Aufwand durch Destillation gereinigt werden. Es hat ein vergleichsweise niedriges Molekulargewicht und ist in den zur Racematspaltung eingesetzten Solventien ausgezeichnet löslich. Nach erfolgreicher Spaltung kann es nach den üblichen Methoden zurückgewonnen und dem Prozess erneut zugeführt werden.

Da die in der Literatur beschriebenen diastereomeren Salze der (−)-Äpfelsäure nur mit Basen gebildet wurden, die grosse Substituenten und damit hohe Molekulargewichte besitzen, ist es im vorliegenden Fall überraschend, dass nach der erfindungsgemässen Umsetzung von D,L-Äpfelsäure mit R(−)-2-Amino-1-butanol eine Gesamtausbeute zwischen 80 und 90% an reinem optisch aktivem Malat erhalten werden kann und zwar weitgehend unabhängig vom verwendeten Lösungsmittel.

Durch die Wahl einer geeigneten Konzentration ist es möglich, ein diastereomeres Salzpaar zur Ausfällung zu bringen, das bereits ohne Umkristallisation eine hohe, für die Weiterverarbeitung geeignete optische Reinheit aufweist. Ein in weitem Konzentrationsbereich wirksames Lösungsmittel ist Methanol, Äthanol oder ein anderer niedermolekularer Alkohol mit möglichst nicht zu hohem Wassergehalt. Aus diesen Lösungsmitteln kristallisiert bevorzugt das (−)(−)-bzw. (+)(+)-Salzpaar.

Bei Einsatz von R(−)-2-Amino-1-butanol kristallisiert z.B. aus einem niedermolekularen Alkohol mit einem Wassergehalt von unter 40, vorzugsweise 20 Gew.%, das (−)(−)-Salzpaar. Die besten Bedingungen für ausreichende Reinheit und Ausbeute können leicht durch Variation der Konzentration ermittelt werden. Die Umsetzung wird vorzugsweise bei Raumtemperatur durchgeführt — das Gemisch erwärmt sich durch exothermen Verlauf der Reaktion — und wird durch anfängliches Rühren zur besseren Durchmischung unterstützt. Animpfen mit (−)(−)-Salz ist zur besseren Kristallisation vorteilhaft. Unter gleichen Bedingungen wird mit S(+)-2-Amino-1-butanol das diastereomere (+)(+)-Salzpaar erhalten. Zur Racematspaltung von je 1 mol D,L-Äpfelsäure kann das optisch aktive 2-Amino-1-butanol in einer Menge zwischen 0,5 und 1 mol Äquivalenten verwendet werden. Bevorzugt werden äquimolare Mengen von Äpfelsäure und 2-Amino-1-butanol verwendet, da auf diese Weise die höchsten Ausbeuten erzielt werden.

*Beispiel 1*

Zu einer Lösung von 1340 g D,L-Äpfelsäure in 1784 ml wasserfreiem Methanol werden bei 25°C 890 g R(−)-2-Amino-1-butanol gegeben. Nach Abklingen der Reaktion und Abkühlen auf Raumtemperatur isoliert man 1015 g festes (91%) R(−)-2-Amino-1-butanol(−)malat ([α]$_D^{25}$ = −7,1, c = 5 g/100 ml in Methanol). Zur Rückspaltung wird das feste Salz mit zwei Moläquivalenten 55%iger Kalilauge versetzt und die entstandene obere Phase, die 2-Amino-1-butanol enthält, von der unteren (Dikaliummalat-)Phase getrennt. Durch Destillation werden 860 g reines R(−)-2-Amino-1-butanol zurückgewonnen. Die wässerige Lösung des Dikaliumsalzes der Äpfelsäure wurde über einen stark sauren Ionenaustauscher in der H⁺-Form (Polystyrolsulfonsäure, verwendet wurde Lewatit S 100, Hersteller Bayer AG) in die freie Säure überführt. Zur Isolierung der L(−)-Äpfelsäure wurde der Ablauf bis auf einen Säuregehalt von 60% im Vakuum eingeengt. Nach Filtration, bei der 9 g unreine (−)-Äpfelsäure gewonnen werden, isoliert man durch Sprühtrocknung 601 g optisch reine L(−)-Äpfelsäure ([a]$_D^{25}$ = 25,7°, c = 5 g/100 ml in Pyridin).

Entsprechend wird die Mutterlauge der Racematspaltung nach Abziehen des Lösungsmittels aufgearbeitet. Beim Einengen der mit dem Ionenaustauscher behandelten Lösung werden zunächst 41 g unreine D(+)-Äpfelsäure isoliert, um anschliessend durch Sprühtrocknung der Lösung 650 g des optisch reinen (+)Antipoden zu gewinnen ([α$_D^{25}$ = 25,3°; c = 5 g/100 ml in Pyridin).

Mit dem erfindungsgemässen Verfahren wird ausserdem bei Einsatz von R(−)-2-Amino-1-butanol reine D(+)-Äpfelsäure gewonnen, indem aus der Mutterlauge der Racematspaltung nach Abziehen des Lösungsmittels zunächst reines R(−)-2-Amino-1-butanol(+)malat ausgefällt, das nach üblichen Methoden in R(−)-2-Amino-1-butanol und D(+)-Äpfelsäure gespalten werden kann. Mit dieser D(+)-Äpfelsäure kann die Spaltung von racemischem Aminobutanol betrieben werden, wobei das wichtige Pharmazwischenprodukt S(+)-2-Amino-1-butanol auf einfache Weise und in besonderer Reinheit gewonnen werden kann.

*Beispiel 2*

Zu einer Lösung von 670 g D(+)-Äpfelsäure in 850 ml Methanol werden bei 25° 445 g D,L-2-Amino-1-butanol gegeben. Nach Abklingen der Reaktion isoliert man bei Raumtemperatur 508 g (91%) S(+)-2-Amino-1-butanol(+)malat ([α]$_D^{25}$ = +7,25, c = 5 g/100 ml in Methanol). Zur Rückspaltung wird die gesamte Salzmenge mit zwei Moläquivalenten 55%iger Kalilauge versetzt und die obere (2-Amino-1-butanol-)Phase von der unteren (Dikaliummalat-)Phase getrennt. Durch Destillation werden 200 g (90%) S(+)-2-Amino-1-butanol ([α]$_D^{20}$ = +9,94°, rein) gewonnen. D(+)-Äpfelsäure wird in Analogie zum Beispiel 1 aus der wässerigen Dikaliummalatphase über einen stark sauren Ionenaustauscher zurückerhalten. Man isoliert dabei 327 g (98%) D(+)-Äpfelsäure ([α]$_D^{25}$ = +25,3°, c = 5 g/100 ml in Pyridin).

Die erhaltenen diastereomeren Salze wurden mit wenig Methanol gewaschen und wiesen danach folgende spezifischen Drehungen auf (in Lösung):

(−)-2-Amino-1-butanol-(−)-malat [α]$_D^{25}$ = 7,3; c = 5 g/100 ml in Methanol

(+)-2-Amino-1-butanol-(+)-malat [α]$_D^{25}$ = +7,25; c = 5 g/100 ml in Methanol

(−)-2-Amino-1-butanol-(+)-malat [α]$_D^{25}$ = 3,4; c = 5 g/100 ml in Methanol

Nach der Aufarbeitung wurden (−)- und (+)-Äpfelsäure mit 97% optischer Reinheit erhalten:

(−)-Äpfelsäure [α]$_D^{25}$ = −25,7° (c = 5 g/100 ml in Pyridin)

(+)-Äpfelsäure [α]$_D^{25}$ = +25,3° (c = 5 g/100 ml in Pyridin)

Für die noch nicht beschriebenen Salze von R(−)- und S(+)-2-Amino-1-butanol mit D(+) und L(−)-Äpfelsäure wurden folgende Stoffdaten ermittelt:

a) R(−)-2-Amino-1-butanol-(−)-malat
Fp. = 115°C; nach Umkristallisation aus Äthanol
[α]$_D^{25}$ = −7,3 (c = 5 g/100 ml in Methanol)
¹H-NMR (D$_2$O): δ(TMS):
   0,90 (t, 3); 1,55 (m,2);
   2,65 (m, 2); 3,12 (m, 1);
   3,6 (m, 2); 4,25 (m, 1)

*Elementaranalyse* für C$_8$H$_{17}$NO$_6$ (223,23):
   Ber.:   C 40,35   H 7,68   N 6,27%
   Gef.:   C 43,01   H 7,62   N 6,30%

b) S(+)-2-Amino-1-butanol(+)malat
Fp. = 115-116°C; nach Umkristallisation aus Äthanol;
[α]$_D^{25}$ = +7,25 (c = 5 g/100 ml in Methanol)
¹H-NMR (D$_2$O): δ(TMS):
   0,90 (t, 3); 1,55 (m, 2);
   2,65 (m, 2); 3,13 (m, 1);
   3,6 (m, 2); 4,25 (m, 1)

*Elementaranalyse* für C$_8$H$_{17}$NO$_6$ (223,26):
   Ber.:   C 43,05   H 7,68   N 6,27%
   Gef.:   C 43,06   H 7,65   N 6,24%

c) R(−)-2-Amino-1-butanol(+)malat
Fp. = 24°C
[α]$_D^{25}$ = −3,4°, c = 5 g/100 ml in Methanol
¹H-NMR (D$_2$O): δ(TMS):
   0,90 (+, 3); 1,55 (m, 2);
   2,65 (m, 2); 3,13 (m, 1);
   3,6 (m, 2); 4,85 (m, 1)

*Elementaranalyse* für C$_8$H$_{17}$NO$_6$ (223,26):
   Ber.:   C 43,05   H 7,68   N 6,27%
   Gef.:   C 43,08   H 7,71   N 6,31%

## Patentansprüche

1. Die diastereomeren Salze von R(−)-2-Amino-1-butanol und S(+)-2-Amino-1-butanol mit D(+)- bzw. L(−)-Äpfelsäure.

2. Verfahren zur Spaltung racemischer Äpfelsäure und Herstellung insbesondere von L(−)-Äpfelsäure durch fraktionierte Kristallisation von diastereomeren Salzen der Äpfelsäure, dadurch gekennzeichnet, dass man D,L-Äpfelsäure mit optisch aktivem 2-Amino-1-butanol in Lösung umsetzt, das auskristallisierende diastereomere Salz von der Mutterlauge abtrennt und anschliessend jeweils die in Kristallisat bzw. Mutterlauge enthaltenen diastereomeren Salze mit Säuren und Basen nach an sich bekannten Methoden jeweils in die optisch aktiven Äpfelsäuren und 2-Amino-1-butanol- rückspaltet.

3. Verfahren nach Anspruch 2, dadurch gekennzeichnet, dass man R(−)-2-Amino-1-butanol verwendet.

4. Verfahren nach Anspruch 2, dadurch gekennzeichnet, dass man als Lösungsmittel einen niedermolekularen Alkohol mit einem Wassergehalt von unter 40 Gew.% verwendet.

5. Verfahren zur Spaltung von racemischem 2-Amino-1-butanol durch fraktionierte Kristallisation von diastereomeren Salzen des 2-Amino-1-butanols, dadurch gekennzeichnet, dass man die Spaltung mit optisch aktiver D(+)-Äpfelsäure unter sinngemässer Umkehrung des Verfahrens nach Anspruch 2 durchführt.

## Claims

1. The diastereomeric salts of R(−)-2-aminobutan-1-ol and S(+)-2-aminobutan-1-ol with D(+)- and L(−)-malic acid.

2. A process for resolving racemic malic acid and in particular preparing L(−)-malic acid by fractional crystallization of diastereomeric salts of malic acid, wherein D,L-malic acid is reacted with optically active 2-aminobutan-1-ol in solution, the diastereomeric salt which crystallizes out is separated from the mother liquor and thereafter the diastereomeric salts respectively contained in the crystals and in the mother liquor are each converted, by means of acids and bases, using conventional methods, to the optically active malic acid and 2-aminobutan-1-ol.

3. A process as claimed in claim 2, wherein R(−)-2-aminobutan-1-ol is used.

4. A process as claimed in claim 2, wherein the solvent used is a low molecular weight alcohol containing less than 40% by weight of water.

5. A process for resolving racemic 2-aminobutan-1-ol by fractional crystallization of diastereomeric salts of 2-aminobutan-1-ol, wherein the resolution is effected with optically active (D(+)-malic acid, using the process according to claim 2 in the converse sense.

## Revendications

1. Les sels diastéréomères de 2-amino-1-butanol R(−) et de 2-amino-1-butanol S(+) avec l'acide malique D(+) ou L(−).

2. Procédé pour le dédoublement d'acide malique racémique et la préparation en particulier d'acide malique L(−) par cristallisation fractionnée de sels diastéréomères de l'acide malique, caractérisé en ce qu'on fait réagir en solution l'acide malique D,L avec un 2-amino-1-butanol optiquement actif, on sépare de la liqueur mère le sel diastéréomère qui s'est séparé à l'état cristallin, puis on redécompose avec des acides et des bases, suivant des procédés en soi connus, les sels diastéréomères contenus respectivement dans le produit de cristallisation et dans la liqueur mère, en les acides maliques optiquement actifs et en 2-amino-1-butanol.

3. Procédé selon la revendication 2, caractérisé en ce qu'on utilise le 2-amino-1-butanol R(−).

4. Procédé selon la revendication 2, caractérisé en ce qu'on utilise, en tant que solvant, un alcool de bas poids moléculaire ayant une teneur en eau de moins de 40% en poids.

5. Procédé pour le dédoublement de 2-amino-1-butanol racémique par cristallisation fractionnée de sels diastéréomères du 2-amino-1-butanol, caractérisé en ce qu'on effectue le dédoublement avec de l'acide malique D(+) optiquement actif, suivant le procédé de la revendication 2, mais en l'inversant.